# EUROPÄISCHE PATENTANMELDUNG

(11) **EP 1 674 105 A1**
(43) Veröffentlichungstag der Anmeldung: **28.06.2006**
(21) Anmeldenummer: 05003727.4
(22) Anmeldetag: 22.02.2005
(51) Int. Cl.: A61K 31/7068, A61J 1/06

(54) **Gebrauchsfertige Gemcitabinlösungen und Gemcitabinlösungskonzentrate**

(30) Priorität: 23.12.2004 DE 102004063347
(71) Anmelder: STADA ARZNEIMITTEL AG, 61118 Bad Vilbel (DE)
(72) Erfinder: Schridde, Edgar, 30451 Hannover (DE); Gimmel, Stefan-Peter, 30655 Hannover (DE); Merbach, Bernd, 30938 Burgwendel (DE)
(74) Vertreter: Hamm, Volker

(57) **Zusammenfassung**

Die Erfindung betrifft pharmazeutische Zusammensetzungen in Form gebrauchsfertiger Zubereitungen von Gemicitabin in wässriger Lösung in einem Behältnis aus Glas. Durch Wahl bestimmter Verhältnisse zwischen benetzter Oberfläche des Behältnisses und dem Volumen der Lösung wurden stabile Lösungen erhalten.

## Beschreibung

Die vorliegende Erfindung betrifft Gemcitabin umfassende pharmazeutische Zusammensetzungen in der Form von gebrauchsfertigen Lösungen.

Gemcitabin (2'-Deoxy-2',2'-difluorcytidin; 1-(4-Amino-2-oxo-1H-pyrimidin-1-yl)-2-deoxy-2,2-difluorribose; dFdC; CAS Nr. 95058-81-4; C₉H₁₁F₂N₃O₄, Mᵣ 263,2) ist eine im Arzneibuch offiziell monographierte Substanz (Official Monographs, USP 27, 1^{st} Supplement USP-NF, page 3060-61 bzgl. "Gemcitabine Hydrochloride" and "Gemcitabine for Injection") und hat die chemische Struktur

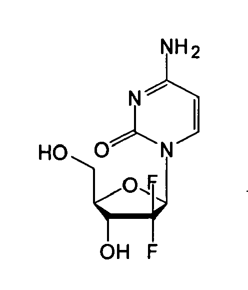

Gemcitabin wurde erstmals in US 4,526,988 beschrieben und kann zur Behandlung von viralen Infektionen oder in der immunosuppressiven Therapie von Autoimmunerkrankungen eingesetzt werden. Die anti-neoplastische Wirksamkeit des Gemcitabins ist in US 5,464,826 offenbart. Es wird beispielsweise allein, aber auch in Kombination mit anderen Zytostatika wie etwa Cisplatin in der Behandlung des lokal fortgeschrittenen oder metastasierten, nichtkleinzelligen Bronchialkarzinoms sowie des fortgeschrittenen Adeno- oder Cysadenokarzinoms des exokrinen Pankreas therapeutisch eingesetzt. Die empfohlene Dosis in der Gemcitabin-Therapie beträgt üblicherweise 1 g / qm Körperoberfläche. Wie andere Nukleosidanaloga kann auch Gemcitabin zytostatisch bei der therapeutischen Behandlung verschiedenster Krebsarten wie z.B. der lymphatischen oder myeloischen Leukämie eingesetzt werden. Dabei erfolgt die Verabreichung von Gemcitabin in der Therapie der verschiedensten Krebserkrankungen intravenös, wodurch es erforderlich wird, den Wirkstoff in Form einer Lösung zur Verfügung zu stellen.

Die zur parenteralen Verabreichung benötigten Gemcitabin-Zubereitungen sind zur Zeit nur in Form von Lyophilisaten (Gemzar®) verfügbar, die vor der Verabreichung an den Patienten rekonstituiert werden müssen. Die Verwendung von solchen gefriergetrockneten Zubereitungen birgt aber wesentliche Nachteile. Zum einen ist das Herstellungsverfahren dieser Lyophilisate kompliziert und teuer, zum anderen stellt die Rekonstitution zusätzliche Arbeitsschritte dar und bedeutet für das damit befasste Personal unerwünschte Risiken. Insbesondere kann es bei der Rekonstituierung der Arzneimittellösungen aus einer Trockensubstanz zum sogenannten "Spray-Back-Effect" kommen, durch den es zu einer weiteren Kontamination und Gefährdung des Personals kommen kann. Demgemäß muss sowohl bei der Herstellung des Lyophilisates, als auch bei seiner Rekonstitution jede Kontamination von Personal oder Inventar mit dem hochwirksamen Zytostatikum vermieden werden. Zudem kann es auch durch andere Fehler in der Handhabung dieser Lyophilisate zu schwerwiegenden Problemen bei der Behandlung mit Gemcitabin kommen, wie z.B. zu einer Abweichung der Wirkstoffkonzentration oder einer mikrobiellen Kontamination der aus dem Lyophilisat hergestellten Lösung.

Aufgrund der vielfältigen Gefahren- und Fehlerquellen bei der Benutzung des lyophilisierten Wirkstoffes zur Herstellung von Gemcitabin-Lösungen war es daher wünschenswert, über gebrauchsfertige, im folgenden auch "ready-to-use-Lösungen" genannte, Arzneimittellösungen zu verfügen.

Bekannterweise sind die gegenwärtig verwendeten, aus Lyophilisaten rekonstituierten Gemcitabin-Lösungen nicht lagerstabil, da sie während ihrer Lagerung einem Abbau des Wirkstoffs unterliegen. Dies führt einerseits zu einer Abweichung der Wirkstoffkonzentration und andererseits zu unerwünschten Verunreinigungen (Abbauprodukten des Gemcitabins) in der Lösung.

Aufgabe der vorliegenden Erfindung ist es damit, stabile, gebrauchsfertige Gemcitabin-Lösungen zur Verfügung zu stellen, die nicht die zuvor diskutierten Risiken und Nachteile der bekannten Darreichungsformen und daraus hergestellter Lösungen aufweisen. In diesem Zusammenhang bedeutet der Begriff "gebrauchsfertig", dass die Lösung nicht unmittelbar vor der Verabreichung an den Patienten aus einem Feststoff, wie etwa einem kristallinen oder amorphen Feststoff oder einem Lyophilisat, rekonstituiert wurde.

Gemcitabinhydrochlorid ist bei Raumtemperatur in stark salzsaurer wässriger Lösung mit ca. 40mg/ml (pH ca. 3; rekonstituiertes GEMZAR-Lyophilisat, Eli Lilly) klar löslich, in neutraler bis schwach alkalischer wässriger Lösung mit ca. 15mg/ml (pH ca. 8) und in alkalischer ethanolisch-wässriger Lösung mit bis zu 110mg/ml (pH ca. 10) klar löslich.

In der veröffentlichten europäischen Patentanmeldung EP 1 479 389 A1 wird das pH-Optimum der Stabilität von Gemcitabin in wässriger Lösung für einen untersuchten pH-Bereich von pH 3 bis 10 mit pH ca. 8 beschrieben.

Da Gemcitabinlösungen in der onkologischen Therapie in einer Dosis von üblicherweise 1000mg/qm Körperoberfläche innerhalb von 30 Minuten infundiert werden, ist es erforderlich, das hierfür notwendige Lösungsvolumen in pharmazeutisch geeigneten Behältnissen zur Verfügung zu stellen.

So werden beispielsweise bei einer zu therapierenden Person mit 180cm Körpergröße und einem Körpergewicht von 80kg und einer damit verbundenen Körperoberfläche von 2qm insgesamt 2000mg Gemcitabin verabreicht.

Damit bieten sich verschiedene Lösungskonzentrationen und Lösungsvolumen an, wie z.B. 200ml Gemcitabinlösung mit einem Gehalt von c=10mg/ml oder 40ml ethanolischwässriges Gemcitabinkonzentrat mit einem Gehalt von c=50mg/ml oder 25ml ethanolischwässriges Gemcitabinkonzentrat mit einem Gehalt von c=80mg/ml, als in geeigneten Packmitteln bereitzustellende Volumen.

Der Wirkstoff Gemcitabin (GEMZAR, Lyophilisat) ist zur Zeit für die Therapie des Bauchspeicheldrüsenkrebses indiziert, desweiteren für die Therapie des Brustkrebses in Kombination mit dem Wirkstoff Paclitaxel als auch für die Therapie des NichtKleinzelligen Lungenkrebses in Kombination mit dem Wirkstoff Cisplatin. Üblicherweise werden diese Wirkstoffkombinationen, als auch andere therapeutische Begleitwirkstofflösungen, nacheinander intravenös über den gleichen Injektionsport verabreicht (sog. Y-Site injection), wobei auf die Kompatibilität der verabreichten Lösungen zueinander geachtet werden muß, um Ausfällungen von Wirkstoffniederschlägen zu vermeiden, da sie als infundierte Partikel lebensbedrohlich sein können. So wurden Informationen zur Kompatibilität von Gemcitabinlösung beispielsweise im "Handbook on Injectable Drugs", 12^{th} Edition, Lawrence A. Trissel, Seite 653-661, im Zusammenhang der Kompatibilität mit verschiedenen Trägerlösungen zur Infusion als auch Kompatibilität mit verschiedensten Wirkstofflösungen (Y-Site Injection Compatibility, 1:1 Mixture) veröffentlicht. Die Gebrauchsinformation GEMZAR selbst macht jedoch keine Aussagen zur Kompatibilität von GEMZAR mit anderen Wirkstoffen.

Jedoch besteht aufseiten der Anwender ein großes Interesse daran, Gemcitabin auch in Kombination mit anderen zytostatisch wirksamen Wirkstoffen in einem so genannten Zytostatikainfusionscocktail (Mischinfusionslösung) zu verabreichen, um hierdurch therapeutisch gewollte Wirksynergismen zu nutzen, und damit die Wirksamkeit der Therapie zu erhöhen, als auch die Patientenbelastung durch zeitaufwendige Einzelinfusionen von zuvor zubereiteten oder gebrauchsfertigen Einzelwirkstofflösungen zu vermeiden, was die Lebensqualität des Patienten verbessern hilft. Desweiteren kann die kombinierte Gabe als Mischinfusion die Möglichkeiten des Therapeuten in der klinischen Praxis im Rahmen seiner Therapiefreiheit deutlich erhöhen, insbesondere im Zusammenhang der Option von zunächst klinischen Prüfungen mit noch heute unbekannten, neuen Wirkstoffen.

Jedoch besitzen diese Wirkstoffe stets individuell zu berücksichtigende, von Gemcitabin abweichende chemische und physikalische Eigenschaften, zum Beispiel hinsichtlich ihrer Löslichkeit, ihrem pH-Stabilitätsoptimum und ihrer physikalisch-chemischen Kompatibilität mit anderen Wirkstoffen. Diese individuellen Eigenschaften wiederum werden insbesondere bestimmt vom pH-Wert in einer wässrigen Lösung.
Im Zusammenhang der Herstellung von Zytostatikainfusionscocktails durch Mischen von Einzelwirkstofflösungen mit ihren unterschiedlichen Eigenschaften, insbesondere voneinander abweichenden pH-Werten, können im Ergebnis physikalisch-chemisch ungünstige pH-Werte resultieren, die beispielsweise eine unerwünschte verringerte Stabilität, unerwünschte Inkompatibilität der Wirkstoffe zueinander oder eine in der Verabreichung für den Patienten lebensbedrohliche Auskristallisation von Partikeln im Lösungsgemisch bewirken können.

Daher kann es in diesem Zusammenhang von Vorteil sein, gebrauchsfertige Gemcitabinlösungen und Gemcitabinkonzentrate mit einem von dem für Gemcitabin als besonders begünstigten pH-Wert von 8 abweichenden pH-Wert zur Verfügung zu stellen, zum Beispiel saurere gebrauchsfertige Gemcitabinlösungen und -konzentrate, beispielsweise mit einem pH-Wert von pH 5, oder zum Beispiel basischere gebrauchsfertige Gemcitabinlösungen und -konzentrate, beispielsweise mit einem pH-Wert von pH 10, so dass o.g. unerwünschte Nachteile im Zusammenhang der Herstellung und Verabreichung von Gemcitabinlösungen allein, oder kombiniert als sogenannte Mischinfusionslösungen, vermieden werden können.

Leider zeigen Gemcitabinlösungen mit einem zu dem besonders begünstigten pH-Wert von pH 8 abweichenden pH-Wert (z.B. pH 5 oder pH 10) deutlich geringere Stabilitäten. Hieraus ergeben sich für die Vermarktung nachteilige, deutlich verkürzte Lagerzeiträume bei der bevorzugten Lagertemperatur von 25°C.

Eine Aufgabe der vorliegenden Erfindung ist es daher, Gemcitabin umfassende gebrauchsfertige Lösungen und Konzentrate zur Verfügung zu stellen, die diese Risiken und Nachteile vermeiden, und die in einem größeren pH-Werte-Bereich Lagerstabilitäten zeigen, die die langfristige Verwendung der gebrauchsfertigen Gemcitabinlösungen und - konzentrate als Therapeutikum erlauben.

Eine weitere Aufgabe der vorliegenden Erfindung ist es, Gemcitabin umfassende gebrauchsfertige Lösungen und Konzentrate zur Verfügung zu stellen, die die o.g. Nachteile vermeiden, indem geeignete lagerstabile Gemcitabinlösungen und -konzentrate mit saureren und basischeren pH-Werten als um pH 8 in hierfür geeigneten Behältnissen bereitgestellt werden.

Eine weitere Aufgabe der vorliegenden Erfindung ist es, charakteristische Glasbehältnismerkmale anzugeben, die wesentliche Voraussetzung dafür sind, dass eine Lagerdauer von 36 Monaten bei 25°C Lagertemperatur erreicht wird, innerhalb der der Wirkstoffgehalt von 95% relativ zum Ausgangsgehalt nicht unterschritten wird.

Gelöst werden diese Aufgaben durch die Merkmale der unabhängigen Ansprüche. Die abhängigen Ansprüche definieren vorteilhafte Ausführungsformen der Erfindung.

In den der vorliegenden Erfindung zugrundeliegenden Experimenten wurde überraschend gefunden, dass Gemcitabin umfassende gebrauchsfertige Lösungen mit einem vom besonders begünstigten pH 8 abweichenden pH-Wert, beispielsweise mit einem pH-Wert von pH 5 oder pH 10, für die Vermarktung besser geeignete Lagerstabilitäten bei 25°C zeigen, wenn sie in dicht verschlossenen Glasbehältnissen, die die in den Ansprüchen beanspruchten Abmessungen einhalten, abgefüllt und gelagert werden.

Diese hierfür geeigneten Glasbehältnisse zeichnen sich durch geometrische Proportionen aus, wobei bevorzugte Behältnisse ein Verhältnis der mit der Wirkstofflösung benetzen Gefäßoberfläche (A) zum Volumen (V) der im Behälter abgefüllten Gemcitabinlösung von weniger als ca. 3,4 (cm²/cm³) aufweisen. Besonders bevorzugt sind solche Gefäße mit einem A/V-Verhältnis von weniger als ca. 2,8, insbesondere weniger als ca. 2,4 (cm²/cm³).

Ebenfalls bevorzugt sind Glasgefäße, bei denen das Verhältnis der Gesamt-GefäßOberfläche (AT) zum Randvollvolumen (RV) kleiner als ca. 4,6 (cm²/cm³) ist. Besonders bevorzugt sind solche Gefäße mit einem AT/RV-Verhältnis von weniger als ca. 4,0 (cm²/cm³), insbesondere weniger als ca. 3,3 (cm²/cm³).

Die erfindungsgemäßen Gefäße sind aus Glas gefertigt, das für Injektabilia geeignet ist. Besonders bevorzugte Packmittel sind im allgemeinen Glasbehältnisse, beispielsweise EN ISO 8362-Glasvial aus Röhrenglas Typ 1, EN ISO 8362-Glasvial aus Hüttenglas Typ 1 oder EN ISO 58363-5 Infusionsflaschen aus Hüttenglas Typ 1.

In der folgenden Tabelle 1 werden beispielsweise das berechnete Verhältnis von Oberfläche zum Volumen (A/V) dargestellt, wobei das Volumen V als das Gemcitabinlösungsvolumen und A als die berechnete, von der Gemcitabinlösung benetzte Glasoberfläche des Glasbehältnisses definiert sind.

**Tabelle 1: Parameter ausgewählter Glasbehältnisse**

| Nennvolumen | ∅ d | Füllhöhe der Lösung | A | A / V |
|---|---|---|---|---|
| V | (cm) | h (cm) | (cm²) | (cm²/cm³) |
| 2ml¹⁾ | 1,60 | 1.00 | 7,04 | 3,52 |
| 4ml¹⁾ | 1,60 | 1,99 | 12,01 | 3,00 |
| 10ml¹⁾ | 2,40 | 2,21 | 21,19 | 2,12 |
| 20ml¹⁾ | 3,00 | 2,83 | 33,74 | 1,97 |
| 30ml²⁾ | 3,60 | 2,95 | 43,54 | 1,45 |
| 50ml³⁾ | 4,25 | 3,52 | 61,19 | 1,22 |
| 100ml²⁾ | 5,16 | 4,78 | 98,40 | 0,98 |
| 250ml³⁾ | 6,60 | 7,31 | 185,73 | 0,74 |

| | | | | |
|---|---|---|---|---|
| ¹⁾ EN ISO 8362-1; Glasvial aus Röhrenglas Typ 1 | | | | |
| ²⁾ EN ISO 8362-1, Glasvial aus Hüttenglas Typ 1 | | | | |
| ³⁾EN ISO 58363-5 Infusionsflaschen aus Hüttenglas Typ 1 | | | | |

In der Tabelle 2 werden beispielsweise das berechnete Verhältnis von Oberfläche zum Volumen (AT/RV) dargestellt, wobei das Volumen RV als das Randvollvolumen des Glasbehältnisses und AT als die berechnete Glasoberfläche des Glasbehältnisses definiert sind.

**Tabelle 2: Parameter ausgewählter Glasbehältnisse**

| Randvollvolumen | ∅ d | Gesamthöhe des Behältnisses | AT | AT / RV |
|---|---|---|---|---|
| RV | (cm) | h (cm) | (cm²) | .cm²/cm³) |
| 2ml¹⁾ | 1,60 | 3,50 | 19,60 | 4,90 |
| 4ml¹⁾ | 1,60 | 4,50 | 24,63 | 4,10 |
| 10ml¹⁾ | 2,40 | 4,50 | 38,45 | 2,85 |
| 20ml¹⁾ | 3,00 | 6,00 | 58,56 | 2,34 |
| 30ml²⁾ | 3,60 | 6,28 | 81,21 | 2,14 |
| 50ml³⁾ | 4,60 | 6,80 | 114,89 | 1,71 |
| 100ml²⁾ | 5,16 | 9,45 | 174,10 | 1,46 |
| 250ml³⁾ | 6,60 | 13,60 | 316,20 | 1,03 |

| | | | | |
|---|---|---|---|---|
| ¹⁾ EN ISO 8362-1; Glasvial aus Röhrenglas Typ 1 | | | | |
| ²⁾ EN ISO 8362-1, Glasvial aus Hüttenglas Typ 1 | | | | |
| ³⁾ EN ISO 58363-5 Infusionsflaschen aus Hüttenglas Typ 1 | | | | |

Der prozentuale Restgehalt an Wirkstoff in der Gemcitabinzubereitung zum Ende der Laufzeit, der aus einem Wirkstoffabbau während der Lagerung resultiert, wird auf ein durch Zulassungsrichtlinien der Arzneimittelbehörden vorgeschriebenes Maß begrenzt mit 95%, relativ zum Ausgangsgehalt.

In diesem Zusammenhang und in den folgenden Stabilitätsuntersuchungen ist der Term "Stabilität der Lösung" immer als Stabilität des Gemcitabins in Lösung zu verstehen, d.h. als langfristige Konstanz der Konzentration der Ausgangsverbindung nach Inlösungbringen.

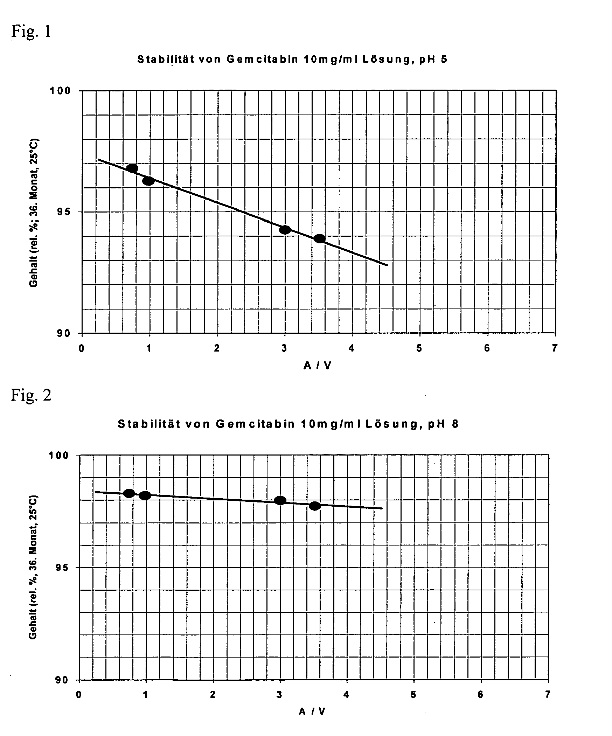

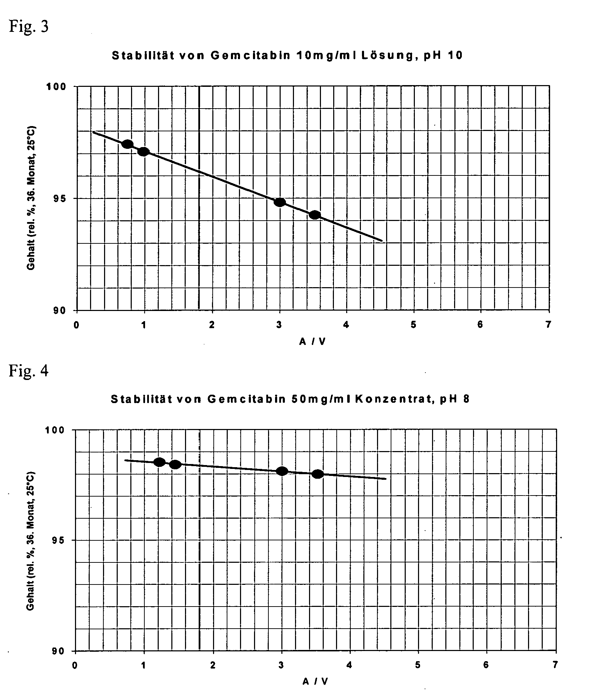

Die nachfolgend näher beschriebenen Stabilitätsuntersuchungen an gebrauchsfertigen Lösungen umfassend Gemcitabin führten zu dem überraschenden Ergebnis, dass Gemcitabin, welches in einer wässrigen Lösung gelöst und in einem dichtverschlossenen Behälter aus Glas abgefüllt ist, eine pharmazeutisch geeignete Stabilität aufweist, wenn dieser Behälter dadurch gekennzeichnet ist, dass das Verhältnis von Gemcitabinlösungbenetzter Oberfläche (A) zu abgefülltem Gemcitabinlösung-Volumen (V) kleiner ist als ca. 3,4 (cm²/cm³). Gefäße mit einem solchen AN-Verhältnis erlauben, wie Fig. 1-5 zu entnehmen ist, die Bereitstellung von langzeit-stabilen, gebrauchsfertigen Gemcitabinlösungen und -lösungskonzentraten; wobei die pH-Werte in einem weiten Bereich von 5 bis 10 liegen können.

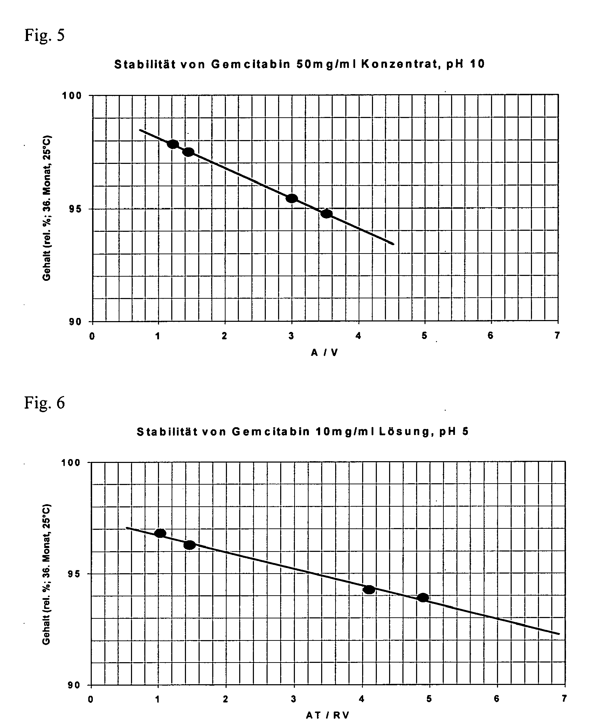

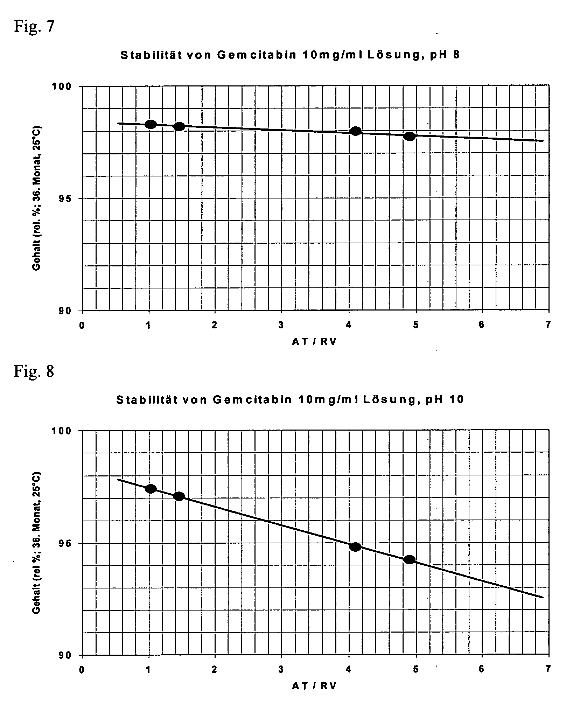

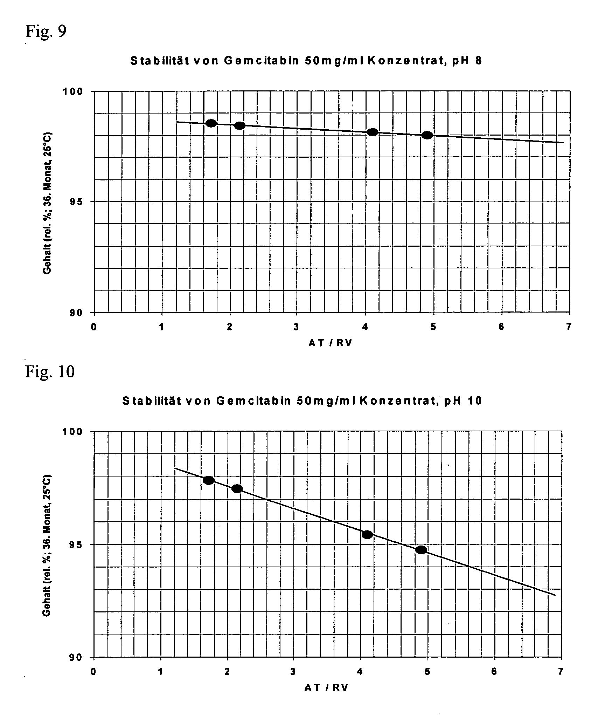

Die Stabilitätsuntersuchungen führten weiterhin zu dem überraschenden Ergebnis, dass Gemcitabin, welches in einer wässrigen Lösung gelöst ist und in einen dichtverschlossenen Behälter aus Glas abgefüllt ist, eine pharmazeutisch geeignete Stabilität aufweist, wenn dieser Behälter dadurch gekennzeichnet ist, dass das Verhältnis von Glasbehältnisoberfläche zum Randvollvolumen kleiner als ca. 4,6 (cm²/cm³, mit AT/RV) ist. Gefäße mit einem solchen AT/RV-Verhältnis erlauben, wie Fig. 7-10 zu entnehmen ist, die Bereitstellung von langzeit-stabilen, gebrauchsfertigen Gemcitabinlösungen und - lösungskonzentraten; wobei die pH-Werte in einem Bereich von 8 bis 10 liegen können. Bei einem AT/RV-Verhältnis von unterhalb 3,3 (cm²/cm³) können, wie aus Fig. 6 ersichtlich, ausreichende Stabilitäten sogar bei einem pH-Wert von 5 erhalten werden.

Für diese völlig überraschend beobachteten Phänomene als Ausdruck einer Abhängigkeit der Stabilität von wässrig gelöstem Gemcitabin von einem geometrischen Parameter des Behältnisses besteht aus dem Stand der Technik bisher kein Hinweis.

Die erfindungsgemäßen Lösungen umfassen zwischen 0,05 mg und 16,0 mg Gemcitabin pro ml Lösungsmittel. Vorzugsweise beträgt die Konzentration des Gemcitabins 10 mg/ml. Dabei können die Lösungen die freie Gemcitabin-Base oder ein physiologisch annehmbares Säureadditionssalz davon umfassen. Bevorzugt wird die freie Gemcitabin-Base, besonders bevorzugt das Säureadditionssalz der Gemcitabin-Base mit einer anorganischen Säure, insbesondere bevorzugt Gemcitabinhydrochlorid zur Herstellung der erfindungsgemäßen Lösungen verwendet. Die erfindungsgemäßen Lösungskonzentrate umfassen zwischen 16,0 mg und 110,0 mg Gemcitabin pro ml Lösungsmittel. Vorzugsweise beträgt die Konzentration des Gemcitabins 50 mg/ml oder 80 mg/ml.

Als geeignete Lösungsmittel für erfindungsgemäße Lösungen und Lösungskonzentrate sind beispielsweise Wasser, Ethanol, Glycerin, 1,2-Propandiol (Propylenglykol), Polyethylenglykol 200-600, Benzylalkohol, Trimethylenglykol, 1,3-Butylenglykol, 2,3-Butylenglykol, Ethylacetat, Ethyllactat, Glykofurol (Tetraglykol), Solketal und Harnstoff zu nennen. Vorzugsweise wird Wasser, Ethanol, Polyethylenglykol 200-600 oder 1,2-Propandiol (Propylenglykol) verwendet, besonders bevorzugt ist Wasser.

Die Einstellung des pH-Wertes der erfindungsgemäßen Lösungen und Lösungskonzentrate kann durch Mischen eines geeigneten Verhältnisses der Gemcitabin-Base mit einem physiologisch annehmbaren Säureadditionssalz davon, vorzugsweise mit Gemcitabinhydrochlorid, erfolgen.

Erfindungsgemäß ist es aber auch möglich, den pH-Wert mit mindestens einem physiologisch annehmbaren Ansäuerungs- und/oder Alkalisierungsmittel einzustellen. Beispielsweise sind hierfür geeignet anorganische Säuren und Basen, wie z. B. Salzsäure, Schwefelsäure, schweflige Säure, Salpetersäure, salpetrige Säure, Phosphorsäure, phosphorige Säure, Kohlensäure, Natriumhydroxid, Kaliumhydroxid, Calciumhydroxid und Magnesiumhydroxid; Alkali- und Erdalkalisalze sowie Alkalihydrogen- und Erdalkalihydrogensalze der anorganischen Oxosäuren des Phosphors, Schwefels, Kohlenstoffs und Stickstoffs wie z. B. Natriumphosphat und dessen Hydrate, Natriumhydrogenphosphat und dessen Hydrate, Dinatriumhydrogenphosphat und dessen Hydrate, Dinatriumsulfat, Natriumhydrogensulfat, Natriumsulfit, Calciumsulfit, Magnesiumsulfit, Calciumhydrogencarbonat, Natriumcarbonat, Natriumhydrogencarbonat, Natriumnitrat, Natriumnitrit, Calciumnitrit, Magnesiumnitrat und Magnesiumnitrit; Salze des Chlors wie z. B. Natriumchlorid, Calciumchlorid und Magnesiumchlorid; organische Basen und Säuren wie z. B. Ameisensäure, Essigsäure, Propionsäure, Milchsäure, Oxalsäure, Malonsäure, Maleinsäure, Weinsäure, Zitronensäure, Brenztraubensäure, Benzoesäure, Methansulfonsäure, Ethansulfonsäure, *para*-Toluol-Sulfonsäure, Salicylsäure, Ascorbinsäure, Tris(hydroxymethyl-)aminomethan (2-Amino-2-(hydroxymethyl)-1,3-propandiol; Trometamol; TRIS), 1-Desoxy-(methylamino)-D-glucitol (N-Methylglucamin; Meglumin); Alkali- und Erdalkalisalze von organischen Basen und Säuren wie etwa Natriumacetat; sowie Mischungen davon.

Vorzugsweise wird der pH-Wert der erfindungsgemäßen Lösungen und Lösungskonzentrate eingestellt mit Salzsäure, Phosphorsäure, Schwefelsäure, Natriumhydroxid, Natriumphosphat und dessen Hydraten, Natriumhydrogenphosphat und dessen Hydraten, Dinatriumhydrogenphosphat und dessen Hydraten, Essigsäure, Milchsäure, Zitronensäure, Methansulfonsäure, Ethansulfonsäure, Tris(hydroxymethyl)aminomethan (Trometamol; TRIS) oder 1-Desoxy-(methylamino)-D-glucitol (N-Methylglucamin; Meglumin), insbesondere bevorzugt mit Natriumhydroxid, Salzsäure, Tris(hydroxymethyl-)aminomethan (Trometamol; TRIS) oder 1-Desoxy-(methylamino)-D-glucitol (N-Methylglucamin; Meglumin).

Die Einstellung und/oder Stabilisierung des pH-Wertes kann auch durch einen aus einem aus physiologisch annehmbaren Ansäuerungs- und/oder Alkalisierungsmittel gebildeten Puffer erfolgen. Besonders bevorzugte Puffer zur Einstellung des pH-Wertes der erfindungsgemäßen Lösungen sind Natriumacetat, Tris(hydroxymethyl-) aminomethan (Trometamol; TRIS), 1-Desoxy-(methylamino)-D-glucitol (N-Methylglucamin; Meglumin) und Dinatriumhydrogenphosphat oder Mischungen davon.

Die erfindungsgemäßen Lösungen und Lösungskonzentrate umfassen gegebenenfalls zusätzlich mindestens einen tonisierenden und/oder mindestens einen konservierenden Hilfsstoff.

Als Tonizitätsmittel für erfindungsgemäße Lösungen und Lösungskonzentrate können beispielsweise verwendet werden physiologisch annehmbare anorganische Alkali- oder Erdalkalisalze wie z. B. Natriumchlorid, Calciumchlorid, Magnesiumchlorid, Natriumsulfat, Natriumcarbonat und Calciumhydrogencarbonat; physiologisch annehmbare organische Salze wie z. B. Natriumlactat; physiologisch annehmbare Kohlenhydrate wie z. B. Glucose, Fructose, Sorbitol, Mannitol, Galactose, Inositol, Maltitol, Lactose, Trehalose, Maltose, Saccharose, Dextran 1, Dextran 10, Dextran 40, Dextran 70, Stärke und Hydroxyethylstärke; physiologisch annehmbare Aminosäuren, Peptide oder Proteine wie etwa Glycin, Albumin und Gelantine; sowie Mischungen davon.

Bevorzugte Tonizitätsmittel sind Natriumchlorid, Calciumchlorid, Glucose, Mannitol und Lactose, insbesondere bevorzugt sind Natriumchlorid, Glucose und Mannitol.

Als Konservierungsmittel für erfindungsgemäße Lösungen und Lösungskonzentrate können beispielsweise verwendet werden Chlorcresol, Benzylalkohol, Ester der *p-*Hydroxybenzoesäure wie etwa Ethylparaben und Methylparaben.

Bevorzugte Konservierungsmittel sind Benzylalkohol, Ethylparaben und Methylparaben, insbesondere bevorzugt ist Benzylalkohol.

Die Herstellung der für die Stabilitätsuntersuchungen erzeugten Prüfmusterchargen mit einer Gemcitabinkonzentration von c=10mg/ml erfolgte in folgender Weise:

Zur Herstellung der unten genannten Lösungen wurden 80 % des benötigten Wassers für Injektionszwecke vorgelegt. Dann wurde Gemcitabinhydrochlorid zugefügt und klar gelöst. Der erwünschte pH-Wert wurde mit den erforderlichen Mengen an 1 N Natronlauge bzw. 1 N Salzsäure (Abweichung maximal +/- 0,2 pH-Stufe) eingestellt.

Die Bestimmung des pH-Wertes erfolgte potentiometrisch. Der Ansatz wurde mit Wasser für Injektionszwecke aufgefüllt und der pH-Wert, wenn erforderlich, nochmals mit den erforderlichen Mengen an 1 N Natronlauge bzw. 1 N Salzsäure eingestellt (Abweichung maximal +/- 0,2 pH-Stufe).

Die Testlösungen wurden sterilfiltriert, jeweils aseptisch in Glasbehältnisse zu deren Nennvolumen abgefüllt, diese mit Durchstechstopfen verschlossen, luftdicht verbördelt und anschließend lichtgeschützt über die Dauer von 12 Monaten bei 25°C und "unter beschleunigten Bedingungen" bei 40°C gelagert.

Der Stabilitätsparameter "pH-Wert" wurde potentiometrisch und der Stabilitätsparameter "Gehalt" per HPLC zu den unten genannten Prüftakten bestimmt. Die Ergebnisse der erfolgten Untersuchungen sind in der nachfolgenden Tabelle 3 zusammengefasst.

Die Analyse der Stabilitätstaktprüfmuster ergab folgende in Tabelle 3 zusammengefasste Ergebnisse.

### Stabilitäten von Gemcitabin 10mg/ml Lösung

### Zusammensetzung:

10mg Gemcitabin (entsprechend 11,39mg Gemcitabinhydrochlorid) gelöst in q.s. Lösungsmittel (Wasser für Injektionszwecke) ad 1,0ml, pH der Lösung eingestellt mit q.s. 1N Natronlauge/1N Salzsäure auf pH 5,0 (+/- 0,2) resp. pH 8,0 (+/- 0,2) resp. pH 10,0 (+/- 0,2)

**Tabelle 3: Resultate der Stabilitätsuntersuchung und extrapolierte Stabilitäten**

| Stärke | **(20mg)** | | | **(40mg**) | | | **(1,0g)** | | | **(2,5g)** | | |
|---|---|---|---|---|---|---|---|---|---|---|---|---|
| Packmittel | **2R**^{**1)**} | | | **4R**^{**1)**} | | | **100H**^{**2)**} | | | **250H**^{**3)**} | | |
| **A / V** | **3,52** | | | **3,00** | | | **0,98** | | | **0,74** | | |
| **AT / RV** | **4,90** | | | **4,10** | | | **1,46** | | | **1,03** | | |

| Gehalt (hplc; rel.%) | **pH 5** | **pH 8** | **pH 10** | **pH 5** | **pH 8** | **pH 10** | **pH 5** | **pH 8** | **pH 10** | **pH 5** | **pH 8** | **pH 10** |
|---|---|---|---|---|---|---|---|---|---|---|---|---|
| Temp. | **25°C** | **25°C** | **25°C** | **25°C** | **25°C** | **25°C** | **25°C** | **25°C** | **25°C** | **25°C** | **25°C** | **25°C** |
| 0mon | 100.0 | 100.0 | 100.0 | 100.0 | 100.0 | 100.0 | 100.0 | 100.0 | 100.0 | 100.0 | 100.0 | 100.0 |
| 3mon | 99.23 | 99.72 | 99.28 | 99.43 | 99.75 | 99.35 | 99.61 | 99.77 | 99.64 | 99.69 | 99.79 | 99.68 |
| 6mon | 98.97 | 99.64 | 99.10 | 99.25 | 99.68 | 99.19 | 99.49 | 99.72 | 99.55 | 99.51 | 99.73 | 99.59 |
| 9mon | 98.25 | 99.31 | 98.26 | 98.27 | 99.39 | 98.43 | 99.03 | 99.45 | 99.12 | 99.06 | 99.49 | 99.22 |
| 12mon | 97.99 | 99.27 | 98.15 | 98.19 | 99.35 | 98.33 | 98.74 | 99.42 | 99.06 | 98.99 | 99.45 | 99.17 |
| **36mon*** | **93.89** | **97.72** | **94.24** | **94.25** | **97.97** | **94.80** | **96.27** | **98.19** | **97.07** | **96.80** | **98.29** | **97.41** |
| **t**_{**95**} **(mon)**** | **29** | **80** | **31** | **31** | **90** | **35** | **48** | **101** | **62** | **56** | **107** | **70** |

| Gehalt (hplc; rel.%) | **pH 5** | **pH 8** | **pH 10** | **pH 5** | **pH 8** | **pH 10** | **pH 5** | **pH 8** | **pH 10** | **pH 5** | **pH 8** | **pH 10** |
|---|---|---|---|---|---|---|---|---|---|---|---|---|
| Temp. | **40°C** | **40°C** | **40°C** | **40°C** | **40°C** | **40°C** | **40°C** | **40°C** | **40°C** | **40°C** | **40°C** | **40°C** |
| 0mon | 100.0 | 100.0 | 100.0 | 100.0 | 100.0 | 100.0 | 100.0 | 100.0 | 100.0 | 100.0 | 100.0 | 100.0 |
| 3mon | 97.54 | 99.19 | 97.96 | 97.78 | 99.28 | 98.16 | 98.75 | 99.36 | 98.97 | 98.89 | 99.40 | 99.08 |
| 6mon | 95.03 | 98.37 | 95.87 | 95.52 | 98.55 | 96.28 | 97.48 | 98.71 | 97.91 | 97.76 | 98.79 | 98.14 |
| 9mon | 92.38 | 97.50 | 93.68 | 93.14 | 97.77 | 94.32 | 96.14 | 98.02 | 96.80 | 96.57 | 98.14 | 97.16 |
| 12mon | 89.16 | 96.44 | 91.00 | 90.23 | 96.84 | 91.89 | 94.51 | 97.18 | 95.45 | 95.12 | 97.35 | 95.95 |
| **36mon*** | **67.98** | **89.49** | **73.42** | **71.15** | **90.66** | **76.07** | **83.79** | **91.67** | **86.56** | **85.59** | **92.18** | **88.05** |
| **t**_{**95**} **(mon)**** | **6** | **17** | **7** | **6** | **19** | **8** | **11** | **22** | **14** | **13** | **23** | **15** |

| | | | | | | | | | | | | |
|---|---|---|---|---|---|---|---|---|---|---|---|---|
| * extrapoliert, | | | | | | | | | | | | |
| ** werte gerundet, (mon = 30Tage) | | | | | | | | | | | | |
| ¹⁾ EN ISO 8362-Glasvial aus Röhrenglas Typ 1 | | | | | | | | | | | | |
| ²⁾ EN ISO 8362-Glasvial aus Hüttenglas Typ 1 | | | | | | | | | | | | |
| ³⁾ EN ISO 58363-5 Infusionsflaschen aus Hüttenglas Typ 1 | | | | | | | | | | | | |

Über die Dauer von 12 Monaten bei einer Lagertemperatur von 25°C und 40°C blieben die Lösungen klar, farblos und frei von sichtbaren Partikeln und Kristallbildung.

Die Herstellung der für die Stabilitätsuntersuchungen erzeugten Prüfmusterchargen mit einer Gemcitabinkonzentration von c=50mg/ml erfolgte in folgender Weise:

Zur Herstellung der in Tabelle 4 genannten Lösungen wurde das Wasser für Injektionszwecke mit 90 % der erforderlichen Menge vorgelegt und Ethanol absolut als ein weiteres Lösungsmittel mit 90 % der erforderlichen Menge zugefügt. Der Ansatz wurde erwärmt. 95 % der erforderlichen Menge an entsprechendem pH-Einstellungsmittel (beispielsweise 5 N Natronlauge/5N Salzsäure) wurden hinzugefügt und der Ansatz wieder erwärmt.

Das Gemcitabinhydrochlorid wurde mit 100 % der erforderlichen Menge hinzugefügt und unter Rühren klar gelöst, wobei der pH-Wert potentiometrisch bestimmt und mit der erforderlichen Menge des entsprechenden Alkalisierungsmittels (beispielsweise 5 N Natronlauge/5N Salzsäure) auf den erwünschten pH-Wert eingestellt wurde.
Der Ansatz wurde mit der erforderlichen Menge an Wasser für Injektionszwecke und dem weiteren Lösungsmittel (Ethanol absolut) aufgefüllt.

Der erwärmte Ansatz wurde bis zum Erhalt einer klaren Lösung gerührt. Der pH-Wert wurde nochmals kontrolliert und erforderlichenfalls mit Natronlauge oder Salzsäure auf den gewünschten pH-Wert eingestellt.
Der Ansatz wurde auf Raumtemperatur abgekühlt und in einen sterilen Behälter sterilfiltriert. Das erhaltene Lösungskonzentrat wurde aseptisch in Glasbehältnisse zu deren Nennvolumen abgefüllt.
Die Durchstechflaschen wurden lichtgeschützt bei 25 °C und "unter beschleunigten Bedingungen" bei 40 °C lichtgeschützt gelagert. Zum jeweiligen Prüftakt wurden Muster der Analyse auf Reinheit (mittels HPLC) und pH-Wert (potentiometrisch) zugeführt.

Die Analyse der Stabilitätstaktprüfmuster ergab folgende in Tabelle 4 zusammengefasste Ergebnisse.

### Stabilitäten von Gemcitabin 50mg/ml Konzentrat

### Zusammensetzung:

50mg Gemcitabin (entsprechend 56,95mg Gemcitabinhydrochlorid) gelöst in q.s. Lösungsmittel (0,500ml Ethanol anhydrous, 0,500 ml Wasser für Injektionszwecke) ad 1,0ml, pH der Lösung eingestellt mit q.s. 5N Natronlauge/5N Salzsäure auf pH 8,0 (+/-0,2) resp. pH 10,0 (+/- 0,2)

**Tabelle 4: Resultate der Stabilitätsuntersuchung und extrapolierte Stabilitäten**

| Stärke | **(100mg)** | | **(200mg)** | | **(1500mg)** | | **(2500mg)** | |
|---|---|---|---|---|---|---|---|---|
| Packmittel | **2R**^{**1)**} | | **4R**^{**1)**} | | **30H**^{**2)**} | | **50H**^{**3)**} | |
| **A / V** | **3,52** | | **3,00** | | **1,45** | | **1,22** | |
| **AT / RV** | **4,90** | | **4,10** | | **2,14** | | **1,71** | |

| Gehalt (hplc; rel.%) | **pH 8** | **pH 10** | **pH 8** | **pH 10** | **pH 8** | **pH 10** | **pH 8** | **pH 10** |
|---|---|---|---|---|---|---|---|---|
| Temp. | 25°C | 25°C | 25°C | 25°C | 25°C | 25°C | 25°C | 25°C |
| 0mon | 100.0 | 100.0 | 100.0 | 100.0 | 100.0 | 100.0 | 100.0 | 100.0 |
| 3mon | 99.80 | 99.60 | 99.86 | 99.65 | 99.82 | 99.76 | 99.92 | 99.85 |
| 6mon | 99.69 | 99.08 | 99.64 | 99.19 | 99.76 | 99.56 | 99.71 | 99.60 |
| 9mon | 99.39 | 98.72 | 99.59 | 98.89 | 99.54 | 99.39 | 99.67 | 99.48 |
| 12mon | 99.37 | 98.25 | 99.35 | 98.47 | 99.49 | 99.14 | 99.51 | 99.28 |
| **36mon*** | **97.98** | **94.75** | **98.12** | **95.42** | **98.42** | **97.48** | **98.53** | **97.83** |
| **t**_{**95**} **(mon)**** | **89** | **34** | **96** | **39** | **115** | **72** | **122** | **83** |

| Gehalt (hplc; rel.%) | **pH 8** | **pH 10** | **pH 8** | **pH 10** | **pH 8** | **pH 10** | **pH 8** | **pH 10** |
|---|---|---|---|---|---|---|---|---|
| Temp. | 40°C | 40°C | 40°C | 40°C | 40°C | 40°C | 40°C | 40°C |
| 0mon | 100.0 | 100.0 | 100.0 | 100.0 | 100.0 | 100.0 | 100.0 | 100.0 |
| 3mon | 99.21 | 98.09 | 99.26 | 98.23 | 99.19 | 99.15 | 99.37 | 98.84 |
| 6mon | 98.14 | 96.73 | 98.67 | 96.12 | 98.93 | 98.07 | 98.81 | 98.07 |
| 9mon | 97.52 | 94.20 | 97.84 | 94.14 | 98.06 | 97.19 | 98.14 | 97.09 |
| 12mon | 96.60 | 91.89 | 96.69 | 91.18 | 97.18 | 95.61 | 97.21 | 96.21 |
| **36mon*** | **89.80** | **76.07** | **91.52** | **74.20** | **91.90** | **87.26** | **91.90** | **88.71** |
| **t**_{**95**} **(mon)**** | **18** | **8** | **21** | **7** | **22** | **14** | **22** | **16** |

| | | | | | | | | |
|---|---|---|---|---|---|---|---|---|
| * extrapoliert, | | | | | | | | |
| ** Werte gerundet, (mon = 30Tage) | | | | | | | | |
| ¹⁾ EN ISO 8362-1; Glasvial aus Röhrenglas Typ 1 | | | | | | | | |
| ²⁾ EN ISO 8362-1, Glasvial aus Hüttenglas Typ 1 | | | | | | | | |
| ³⁾ EN ISO 58363-5 Infusionsflaschen aus Hüttenglas Typ 1 | | | | | | | | |

Über die Dauer von 12 Monaten bei einer Lagertemperatur von 25°C und 40°C blieben die Lösungen klar, farblos und frei von sichtbaren Partikeln und Kristallbildung.

### Beispiele 1-3

Es wurden wässrige Lösungen folgender Zusammensetzung hergestellt:

| | Beispiel 1 | Beispiel 2 | Beispiel 3 |
|---|---|---|---|
| Stärke | 500mg | 1000mg | 2000mg |
| | (50ml) | (100ml) | (200ml) |
| Glasbehältnis | 50H²⁾ | 100H²⁾ | 250ml³⁾ |
| Gemcitabin HCl | 10mg/ml | 10mg/ml | 10mg/ml |
| pH-Wert | 5,5 (+/-0,2) | 8,0 (+/-0,2) | 9,5 (+/-0,2) |
| Natronlauge 1N | q.s. ad pH 5,5 (+/-0,2) | q.s. ad pH 8,0 (+/-0,2) | q.s. ad pH 9,5 (+/-0,2) |
| Salzsäure 1N | q.s. ad pH 5,5 (+/-0,2) | q.s. ad pH 8,0 (+/-0,2) | q.s. ad pH 9,5 (+/-0,2) |
| Trometamol | - | - | 0,20mg/ml |
| Natriumazetat | 0,20mg/ml | - | - |
| Natriumchlorid | 6,5mg/ml | 6,5mg/ml | 6,5mg/ml |
| Wasser für Injektionszwecke | ad 1ml | ad 1ml | ad 1ml |

| | | | |
|---|---|---|---|
| ¹⁾ EN ISO 8362-1; Glasvial aus Röhrenglas Typ 1 | | | |
| ²⁾ EN ISO 8362-1, Glasvial aus Hüttenglas Typ 1 | | | |
| ³⁾ EN ISO 58363-5 Infusionsflaschen aus Hüttenglas Typ 1 | | | |

### Beispiele 4-6

Es wurden wässrige Lösungskonzentrate folgender Zusammensetzung hergestellt:

| | Beispiel 4 | Beispiel 5 | Beispiel 6 |
|---|---|---|---|
| Stärke | 500mg | 1000mg | 2000mg |
| | (10ml) | (20ml) | (40ml) |
| Glasbehältnis | 10R¹⁾ | 20R¹⁾ | 50H²⁾ |
| Gemcitabin HCl | 50mg/ml | 50mg/ml | 50mg/ml |
| pH-Wert | 7,0 (+/-0,2) | 7,5 (+/-0,2) | 9,0 (+/-0,2) |
| Natronlauge 5N | q.s. ad pH 7,0 (+/-0,2) | q.s. ad pH 7,5 (+/-0,2) | q.s. ad pH 9,0 (+/-0,2) |
| Salzsäure 5N | q.s. ad pH 7,0 (+/-0,2) | q.s. ad pH 7,5 (+/-0,2) | q.s. ad pH 9,0 (+/-0,2) |
| Natriumazetat | 0,40mg/ml | - | - |
| 50Vol% Ethanol in Wasser für Injektionszwecke | ad 1ml | ad 1ml | ad 1ml |

| | | | |
|---|---|---|---|
| ¹⁾ EN ISO 8362-1; Glasvial aus Röhrenglas Typ 1 | | | |
| ²⁾ EN ISO 8362-1, Glasvial aus Hüttenglas Typ 1 | | | |
| ³⁾EN ISO 58363-5 Infusionsflaschen aus Hüttenglas Typ 1 | | | |

## Patentansprüche

1. Pharmazeutische Zusammensetzung umfassend eine gebrauchsfertige Zubereitung von Gemcitabin in wässriger Lösung in einem Behältnis aus Glas,
**dadurch gekennzeichnet, dass** das Verhältnis der Oberfläche des Behälters, die von der Zusammensetzung benetzt wird, und dem Volumen der im Behälter aus Glas enthaltenen Zusammensetzung, ausgedrückt in cm²/cm³, kleiner ist als 3,4, und die gebrauchsfertige Zusammensetzung nicht unmittelbar vor der Verabreichung an den Patienten aus einem Feststoff rekonstituiert wurde.

2. Pharmazeutische Zusammensetzung umfassend eine gebrauchsfertige Zubereitung von Gemcitabin in wässriger Lösung in einem Behältnis aus Glas,
**dadurch gekennzeichnet, dass** das Verhältnis von Glasbehälteroberfläche zum Glasbehälterrandvollvolumen, ausgedrückt in cm²/cm³, kleiner ist als 4,6, und die gebrauchsfertige Zusammensetzung nicht unmittelbar vor der Verabreichung an den Patienten aus einem Feststoff rekonstituiert wurde.

3. Pharmazeutische Zusammensetzung nach Anspruch 2, wobei das Verhältnis von Glasbehälteroberfläche zum Glasbehälterrandvollvolumen, ausgedrückt in cm²/cm³, kleiner ist als 3,3.

4. Pharmazeutische Zusammensetzung nach einem der Ansprüche 1-3, wobei der Behälter aus Hüttenglas Typ 1 gefertigt ist.

5. Pharmazeutische Zusammensetzung nach einem der Ansprüche 1-3, wobei der Behälter aus Röhrenglas Typ 1 gefertigt ist.

6. Pharmazeutische Zusammensetzung nach einem der Ansprüche 1-5, wobei die Gemcitabin-Konzentration zwischen 0,05 mg/ml und 110 mg/ml liegt.

7. Pharmazeutische Zusammensetzung nach Anspruch 6, wobei die Gemcitabin-Konzentration 5 mg/ml oder 80 mg/ml beträgt.

8. Pharmazeutische Zusammensetzung nach einem der Ansprüche 1-7, wobei der pH-Wert in einem Bereich von ca. 5,0 bis ca. 10,0 liegt.

9. Pharmazeutische Zusammensetzung nach einem der Ansprüche 1-8, die gegebenenfalls zusätzlich mindestens einen tonisierenden und/oder mindestens einen konservierenden Hilfsstoff umfasst.

10. Verwendung der pharmazeutischen Zusammensetzung nach einem der Ansprüche 1-9 zur Herstellung einer Lösung zur Verabreichung auf parenteralem Weg.

11. Verwendung der pharmazeutischen Zusammensetzung nach einem der Ansprüche 1-10 zur Herstellung eines Arzneimittels zur Behandlung einer Tumorerkrankung.
